# EUROPEAN PATENT APPLICATION

(11) **EP 2 876 165 A1**
(43) Date of publication of application: **27.05.2015**
(21) Application number: 13194010.8
(22) Date of filing: 22.11.2013
(51) Int. Cl.: C12P 21/02, C12N 1/38

(54) **Improved method for producing modifed target polypeptides**

(71) Applicant: ACIB GmbH, 8010 Graz (AT); Universität für Bodenkultur Wien, 1190 Vienna (AT)
(72) Inventor: Wiltschi, Birgit, 8042 Graz (AT); Fladischer, Patrik, 8020 Graz (AT); Anderhuber, Nikolaus, 79787 Lauchringen (DE); Striedner, Gerald, Wien 1220 (AT); Mairhofer, Jürgen, 1170 Wien (AT)
(74) Representative: Gassner, Birgitta

(57) **Abstract**

The present invention relates to an enhanced method for producing a modified target polypeptide containing at least one non-canonical amino acid. In particular, the present invention relates to an improved fermentation method for producing a modified target polypeptide containing at least one non-canonical amino acid, wherein the host cell is cultivated in a minimal medium comprising a complex amino acid source with a limiting amount of at least one canonical amino acid.

## Description

The present invention relates to an enhanced method for producing a modified target polypeptide containing at least one non-canonical amino acid. In particular, the present invention relates to an improved fermentation method for producing a modified target polypeptide containing at least one non-canonical amino acid, wherein the host cell is cultivated in a minimal medium comprising a complex amino acid source with a limiting amount of at least one canonical amino acid.

### BACKGROUND OF THE INVENTION

Naturally occurring proteins and enzymes display highly diverse functionalities that are perfectly adapted to operate in their natural environment. Wild-type enzymes, though, do not always exhibit the properties needed to make them useful biocatalysts for industrial application.

The 20 canonical amino acids (cAA) encoded by the genetic code furnish proteins with a limited variety of chemical groups being responsible for limitations of these approaches. Nature compensates these limitations by introducing chemical diversity via post-translational modifications (J. Seo and K.J. Lee, J Biochem Mol Biol. 2004 Jan 31; 37(1):35-44). In contrast, non-canonical amino acids (ncAAs) are not encoded by the genetic code. Yet, they are very abundant in nature and display much more diverse side chain chemistries than the cAAs. Permitting ncAAs for ribosomal translation into proteins vastly expands the panoply of chemical protein modifications (S. Zheng and I. Kwon, Biotechnol J. 2012 Jan; 7(1):47-60).

Existing protocols for the incorporation of non canonical amino acids are based on the use of auxotrophic strains. The current protocols employ either a medium shift from complex LB medium to synthethic minimal medium or use a complex synthetic multi-component medium. Both approaches render up-scaling very unpractical and lower reproducibility.

US20100273978 discloses compositions and methods of identifying, modifying and producing modified target molecules, including therapeutic molecules by modification with non-natural amino acids. Auxotrophic host cells are grown in a complex culture medium until a pre-determined concentration. Thereafter, the complex culture media is replaced by a minimal medium lacking at least one cAA to which defined amounts of non-natural amino acids are added.

WO2007103307 describes a method for incorporating ncAA into a polypeptid or protein by utilizing a mutant or modified aminoacyl-tRNA synthetase. The host cells are grown on an M9-minimal medium until a predetermined concentration. Thereafter, non-natural amino acids are added to the culture medium.

WO2003073238 discloses a method to incorporate unnatural amino acid analogs into protein products to generate proteins of modified or novel functions. The host cells are grown on an M9-minimal medium until a predetermined concentration. Thereafter, non-natural amino acids analogs are added to the culture medium.

In US20020042097 methods for producing modified polypeptides containing amino acid analogs are disclosed. The host cells are grown on an M9AA-minimal medium.

EP0992586 discloses the incorporation of certain amino acid analogs into polypeptides produced by cells. The degree of incorporation can be regulated by adjusting the concentration of amino acid analogs in the media and/or by adjusting osmolality of the media.

However, a reliable process for a large-scale production of modified target polypeptides has not yet been described.

### SUMMARY OF THE INVENTION

It is the objective of the present invention to provide an enhanced method for producing a modified target polypeptide containing at least one non-canonical amino acid (ncAA) comprising the steps of:
a) providing a host cell, the host cell comprising a vector encoding a target polypeptide;
b) cultivating said host cell in a medium comprising a complex amino acid source with a limiting amount of at least one canonical amino acid (cAA), whereupon said host cell logarithmically grows until depletion of said cAA;
c) adding a corresponding ncAA to said medium and expressing the target protein, whereupon the ncAA is incorporated into the target polypeptide; and
d) isolating the modified target polypeptide.

A further aspect of the invention is the method as described above, wherein the host cell is a bacterial cell, preferably from the *Enterobacteriaceae* family, more preferred an *Escherichia* cell.

A further aspect of the invention is the method as described above, wherein the host cell is auxotrophic for at least one canonical amino acid.

A further aspect of the invention is the method as described above, wherein the complex amino acid source is a hydrolysate of protein material that is rich in amino acids, nitrogen compounds, carbohydrate and vitamins, such as yeast extract, casamino acids, tryptone, beef extract, malt extract, soy peptone, or lactalbumin hydrolysate, preferably it is a yeast extract.

A further aspect of the invention is the method as described above, wherein the complex amino acid source is added to a minimal medium.

A further aspect of the invention is the method as described above, wherein said ncAA is selected from the group consisting of methionine analog, tryptophane analog or trypsine analog. In a further embodiment of the invention the ncAA is selected from the group consisting of methoxinine, norleucine, homopropargylglycine, meta-fluoro-phenylalanine, ortho-fluoro-phenylalanine, para-fluoro-phenylalanine, cis-hydroxy-proline, trans-hydroxy-proline, cis-fluoro-proline, trans-fluoro-proline, 4-aza-tryptophane, 4-fluoro-tryptophane, 7-fluoro-tryptophane, 4-amino-trypthophane, 4-thiofuran-tryptophane, meta-fluoro-tyrosine, ortho-fluorotyrosine, dopamine, preferably the ncAA is norleucine or a tryptophane analog.

A further aspect of the invention is the method as described above, wherein the modified target polypeptide is selected from the group consisting of antibodies, antibody fragments, enzymes, receptors, hormones, regulatory factors, growth factors, antigens, membrane proteins and binding agents.

A further aspect of the invention is the method as described above, wherein the method is carried out in a bioreactor.

A further aspect of the invention is the method as described above, wherein the bioreactor is a large-scale bioreactor.

A further aspect of the invention is the method as described above, wherein the bioreactor has a capacity of about 500 liters.

A further aspect of the invention is an enhanced method for substitution of a canonical amino acid by a ncAA during translation of a target polypeptide in a host cell, comprising culturing said cell in minimal growth medium and a defined amount of a complex amino acid source until a defined point in the growth curve, then adding the desired ncAA directly to the culture media, whereupon the ncAA is incorporated into the target polypeptide.

A further aspect of the invention is the method as described above, wherein the complex amino acid source is a yeast extract, casamino acids, tryptone, beef extract, malt extract, soy peptone, or lactalbumin hydrolysate, preferably it is a yeast extract.

A further aspect of the invention is the method as described above, wherein the yeast extract concentration is in the range of about 0.5 to 10 g/L, preferably in the range of about 1 to 8 g/L, more preferred in the range of about 2 to 6 g/L.

A further aspect of the invention is an enhanced method for substitution of a canonical amino acid by a ncAA during translation of a target polypeptide in a host cell, comprising
a) providing a genetically engineered host cell which is capable of producing the ncAA, said cell comprising a vector encoding the target polypeptide, and
b) culturing said cell in minimal growth media and a defined amount of complex amino acids source whereupon the ncAA is biosynthesized, and
c) inducing expression of the target polypeptide, whereupon the biosynthesized ncAA is incorporated into the target polypeptide, and
d) isolating the target polypeptide.

A further aspect of the invention is a method for designing a growth medium for producing a modified target polypeptide comprising:
a) adding defined amounts of a complex amino acids source to a minimal medium;
b) growing a host cell in said medium;
c) measuring the D₆₀₀ of said medium at repeated intervals over a time period;
d) correlating the amounts of the complex amino acids source added and the arrest of growth;
e) supplementing the minimal medium with the determined amount of the complex amino acids source.

### BRIEF DESCRIPTION OF THE DRAWINGS

Fig. 1: Yest extract dependent growth of the Met-auxothrophic strain B834(DE3) carrying {pTTL} in 100 mL shake flask cultures in M9 medium containing 1 g/L (black circle), 2 g/L (black square), 3 g/L (black diamond), 4 g/L (white circle), and 6 g/L (white diamond) YE in comparison to 1 g/L YE + supplementation with excess Met (white square). D₆₀₀ values higher than 3.6 were normalized to CDM.
Fig. 2: Correlation of cell dry mass (CDM) and optical density (D₆₀₀)
   A linear correlation between D₆₀₀ and CDM was observed for D₆₀₀ ≤3.6 for a culture supplemented with 1 g/L YE and excess Met (10 mM).
Fig. 3: Expression of TTL[Met] and TTL[Nle] in 100 mL cultures
   10% SDS polyacrylamide gel; Comassie blue stain. The size of the relevant molecular weight marker bands is indicated on the left gel margin. M, pre-stained protein ladder mix; ni, non-induced culture; i, induced culture; -, empty lane. In the induced sample a black trianglered arrow) is detectable. In addition, a second smaller unknown protein is present in the induced samples (white triangle).

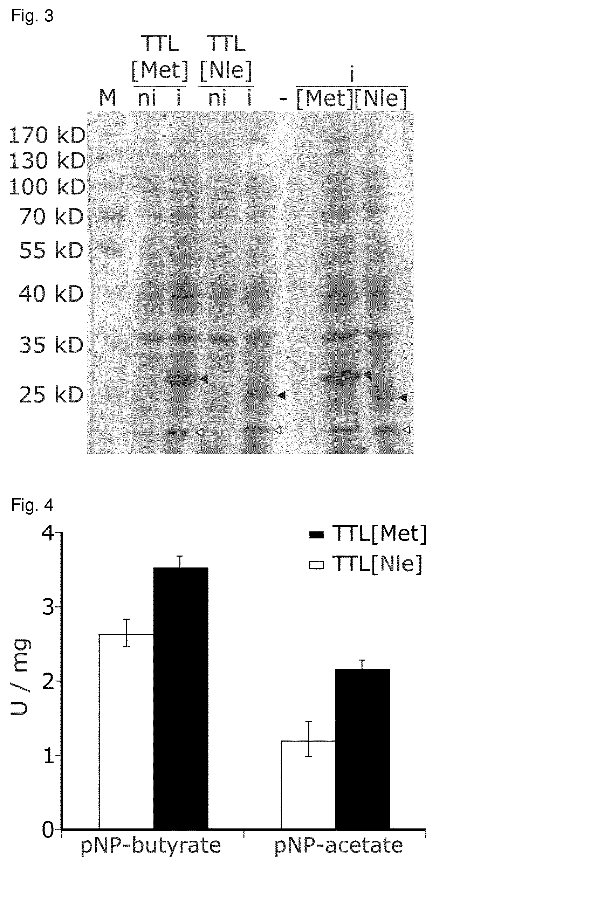

Fig. 4: Increased activity of purified synthetic TTL on standard esterase substrates.

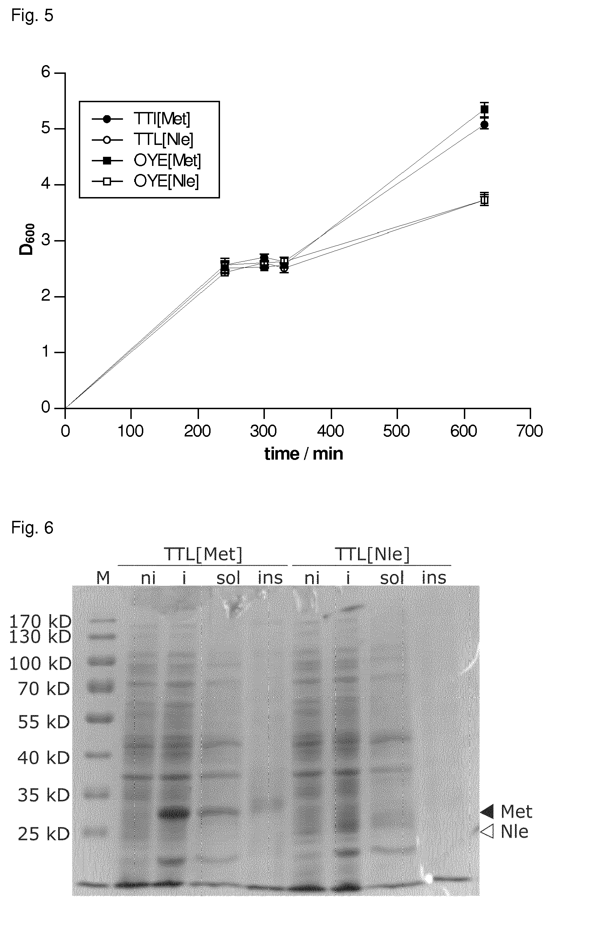

Fig. 5: Growth curves of strain B834(DE3) expressing TTL or OYE in shake flask experiments. The attenuance at 600 nm (D₆₀₀) vs. time is shown.
Fig. 6: Expression of synthetic TTL and OYE in shake flask cultures. 10% SDS polyacrylamide gel; Comassie blue stain. M, prestained SDS ladder mix (5 µL); ni, non-induced; i, induced after 5 hours, sol, soluble protein fraction; ins, insoluble protein fraction.

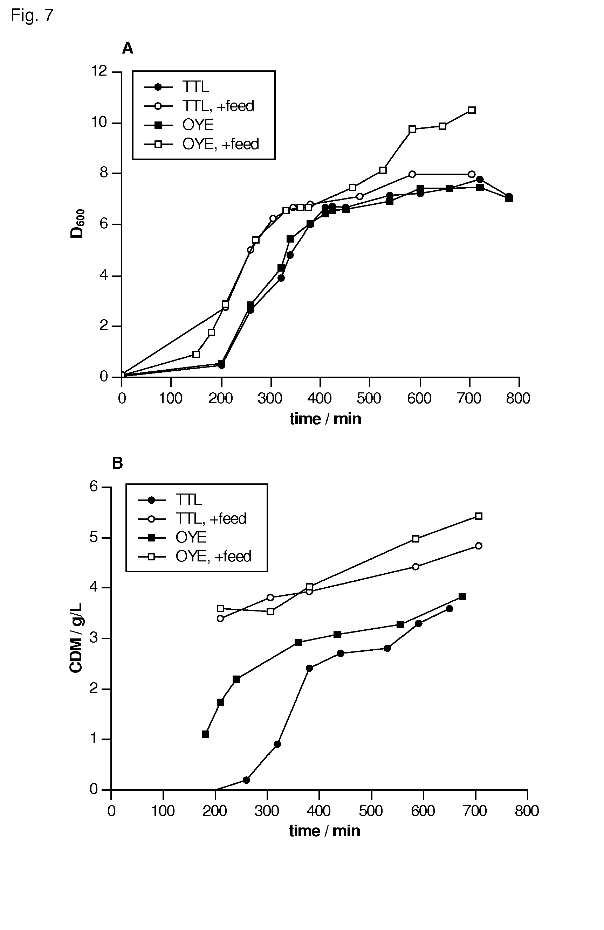

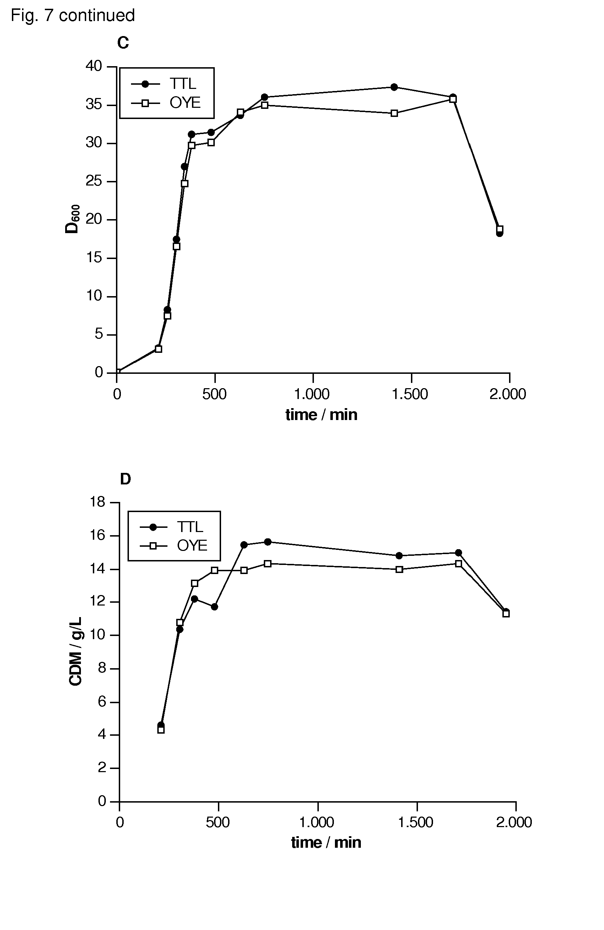

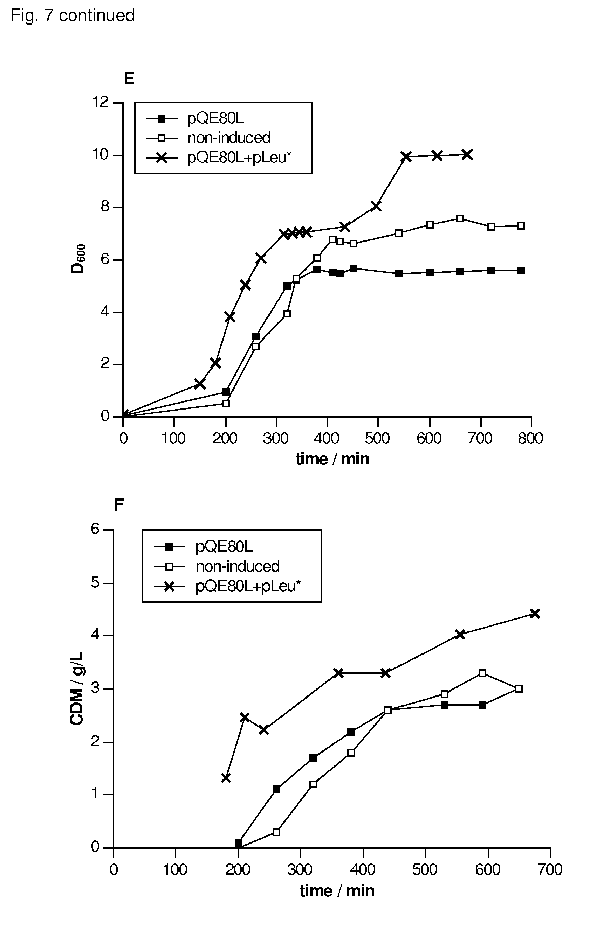

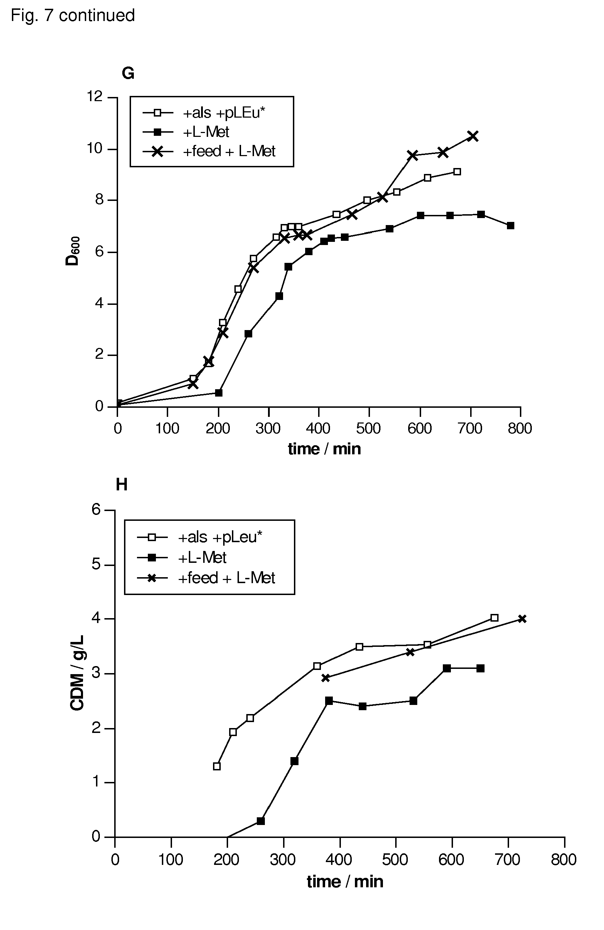

Fig. 7: Bioprocess analysis for the incorporation of biosynthesized Nle into TTL or OYE.
   A: Growth curves D₆₀₀ vs. time for BWEC14 {pQE80L-TTL-h6} and BWEC14 {pEamTA-OYE}.
   B: Growth curves cell dry mass (CDM) vs. time for BWEC14 {pQE80L-TTL-h6} and BWEC14 {pEamTA-OYE}.
   C: Growth curves D₆₀₀ vs. time for BWEC14 {pQE80L-TTL-h6} and BWEC14 {pEamTA-OYE} with increased cell density of D₆₀₀ of 30.
   D: Growth curves cell dry mass (CDM) vs. time for BWEC14 {pQE80L-TTL-h6} and BWEC14 {pEamTA-OYE} with increased cell density of D₆₀₀ of 30.
   E: The D₆₀₀ for a non-induced culture (non-induced), BWEC14 carrying the empty plasmid (pQE80L) and BWEC14 carrying the empty, as well as the pLeuA* overexpression plasmid (D₆₀₀ 6 pQE80L + pLeuA*) are also shown.
   F: CDM values for a non-induced culture (non-induced), BWEC14 carrying the empty plasmid (pQE80L) and BWEC14 carrying the empty, as well as the pLeuA* overexpression plasmid (D₆₀₀ 6 pQE80L + pLeuA*) are also shown.
   G: The D₆₀₀ for the als + strain B834(DE3) carrying the pLeuA* overexpression plasmid (+als +pLeu*), as well as the controls where the L-Met (cAA) was added at the time of induction for the process with (+feed, +Met) and without (+L-Met) additional nutrient feed during protein induction, is shown.H: The CDM for the als + strain B834(DE3) carrying the pLeuA* overexpression plasmid (+als +pLeu*), as well as the controls where the L-Met (cAA) was added at the time of induction for the process with (+feed, +Met) and without (+L-Met) additional nutrient feed during protein induction, is shown.

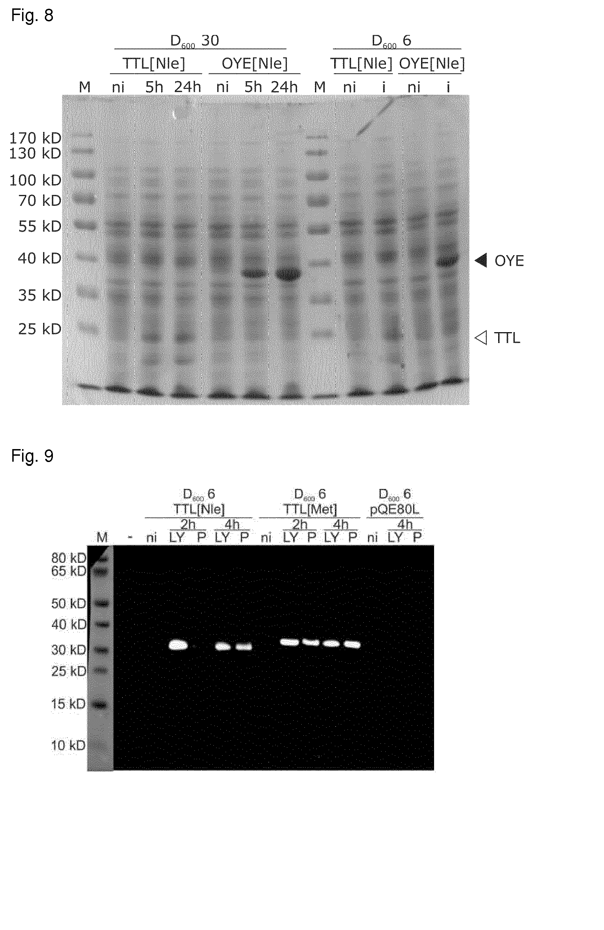

Fig. 8: Expression of TTL and OYE in the presence of biosynthesized Nle. 10% SDS polyacrylamide gel; Comassie blue stain. M, prestained SDS ladder mix (5 µL); ni, non-induced; i, induced after 5 hours and 24 hours. Two different scales, D₆₀₀ 6 and D₆₀₀ 30 are shown.
Fig. 9: Time course of TTL[Met] and TTL[Nle] expression. Western Blot of soluble (LY) and insoluble (P) protein fractions of non-induced (ni) samples compared to samples after 2 h and 4 h of induction with 1 mM IPTG are shown. The pQE80 sample contained an empty expression vector for control. The transmission image of the molecular weight standard (M) is shown.

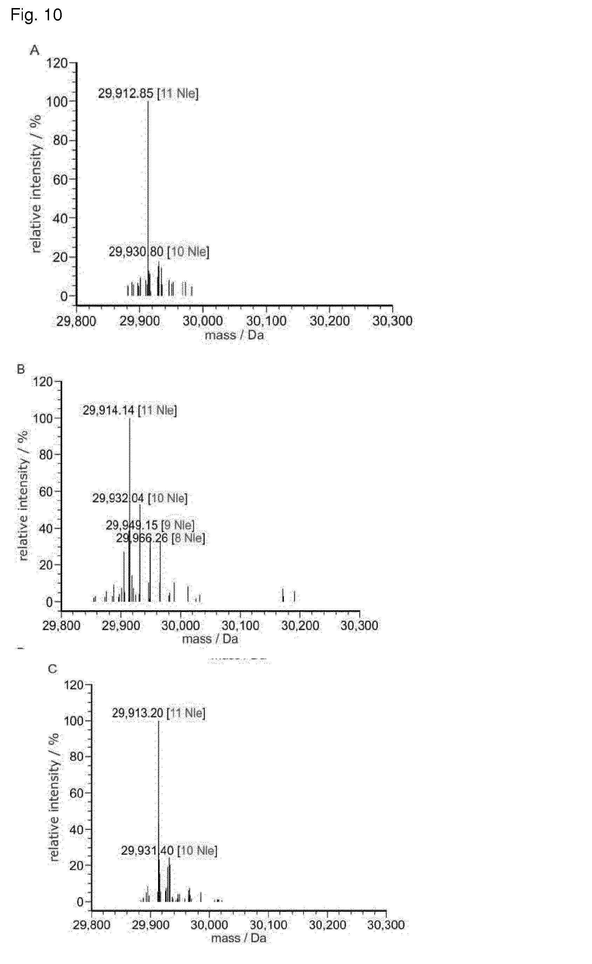

Fig. 10: Determination of the incorporation efficiency of Nle into TTL. LC-ESI MS-spectra of TTL[Nle] produced by the incorporation of biosynthesized Nle at D₆₀₀ 6 without feed (A), D₆₀₀ 6 with feed (B) and D₆₀₀ 30 (C) are shown.

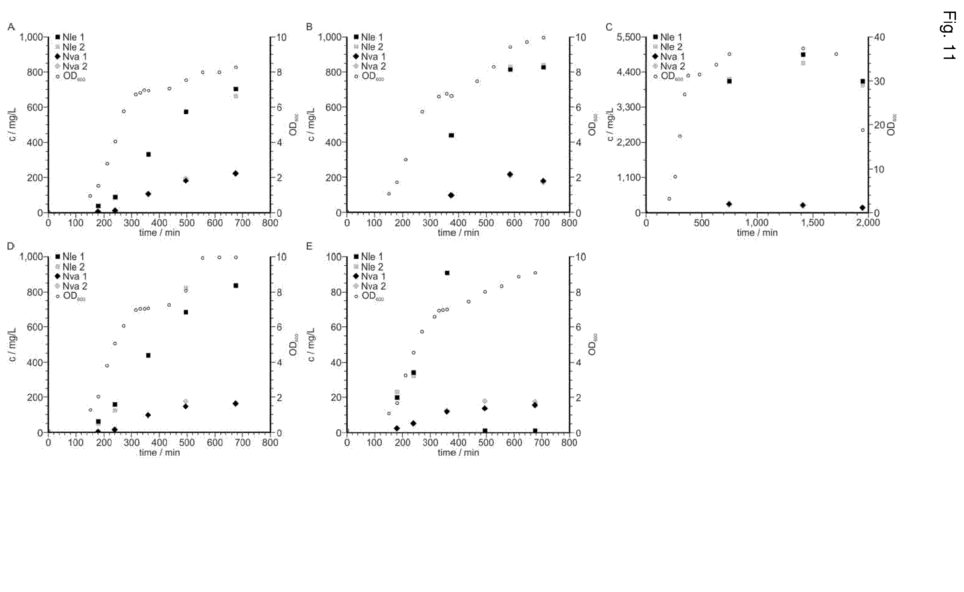

Fig. 11: Determination of Nva and Nle productivity of BWEC14. The productivity of the two ncAA Nle (black and grey square) and Nva (black and grey diamond) was followed over time. The D₆₀₀ (white circles) is shown to demonstrate that Nle and Nva were produced during growth and not in resting cells.

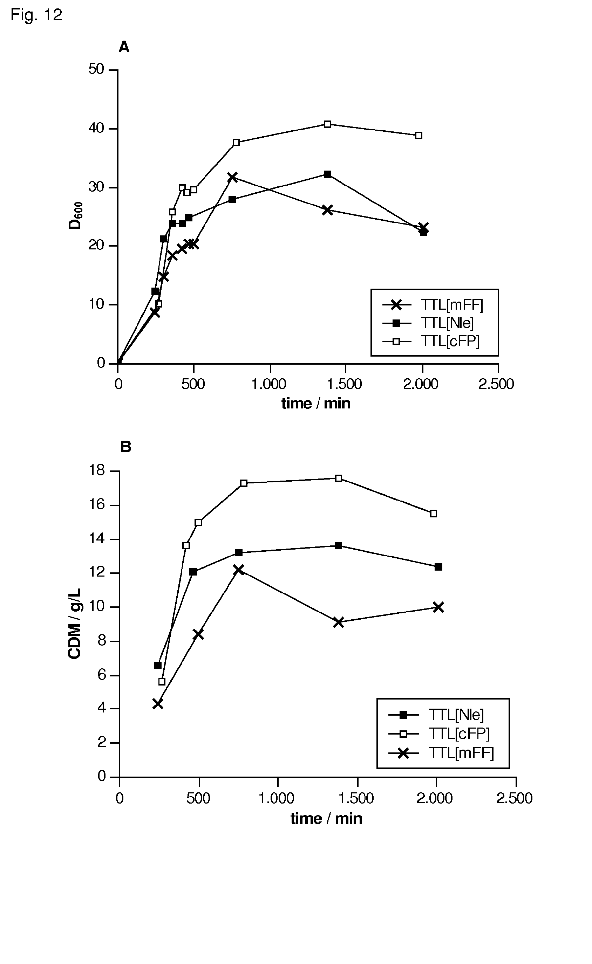

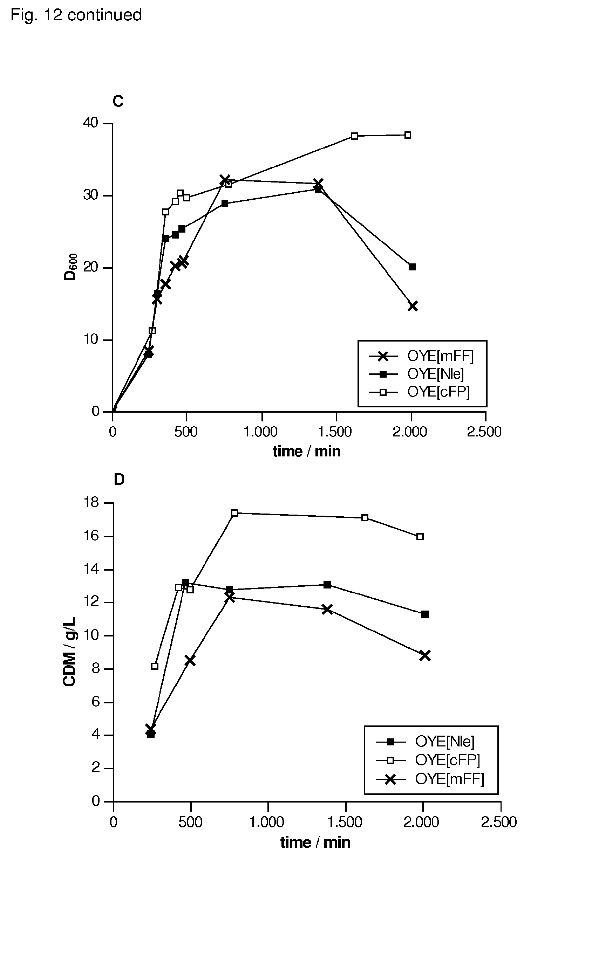

Fig. 12: Growth curves D₆₀₀/CDM of the bioreactor processes. The Met auxotrophic strain B834(DE3), the Pro auxotrophic strain BWEC44 and the Phe auxotrophic strain BWEC23 were used for the incorporation of either Nle (Met analog), cFP/tFP (Pro analogs) or mFF (Phe analog).
   A: Growth curves D₆₀₀ vs. time for the strains carrying the pQE80L-TTL-h6 expression plasmid.
   B: Growth curves CDM vs. time for the strains carrying the pQE80L-TTL-h6 expression plasmid.
   C: Growth curves D₆₀₀ vs. time for the strains carrying the pEamTA-OYE expression plasmid.
   D: Growth curves CDM vs. time for the strains carrying the pEamTA-OYE expression plasmid.

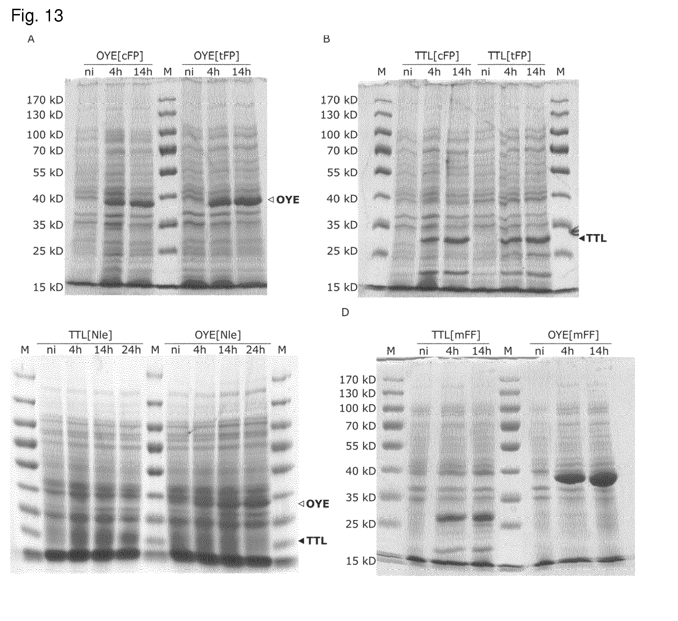

Fig. 13: Expression of synthetic TTL and OYE variants by ncAA supplementation in the bioreactor. 10% SDS polyacrylamid gel; Comassie blue stain. M, prestained SDS ladder mix (5 µL); ni, non-induced; i, induced after 4 h, 14 h and 24 h.
   A: An overexpression band for OYE[cFP] and OYE[tFP] is visible at the expected size of 37 kD (white mark).
   B: An overexpression band for TTL[cFP] and TTL[tFP] is visible at the expected size of 30 kD (black mark).
   C: An overexpression band for the OYE[Nle] is clearly visible at the expected size of 37 kD (white mark), as well as for TTL[Nle] at 25 kD (black mark) as expected.
   D: An overexpression band for the OYE[mFFe] is clearly visible at expected size of 37 kD (white mark), as well as for TTL[Nle] at 30 kD (black mark) as calculated.

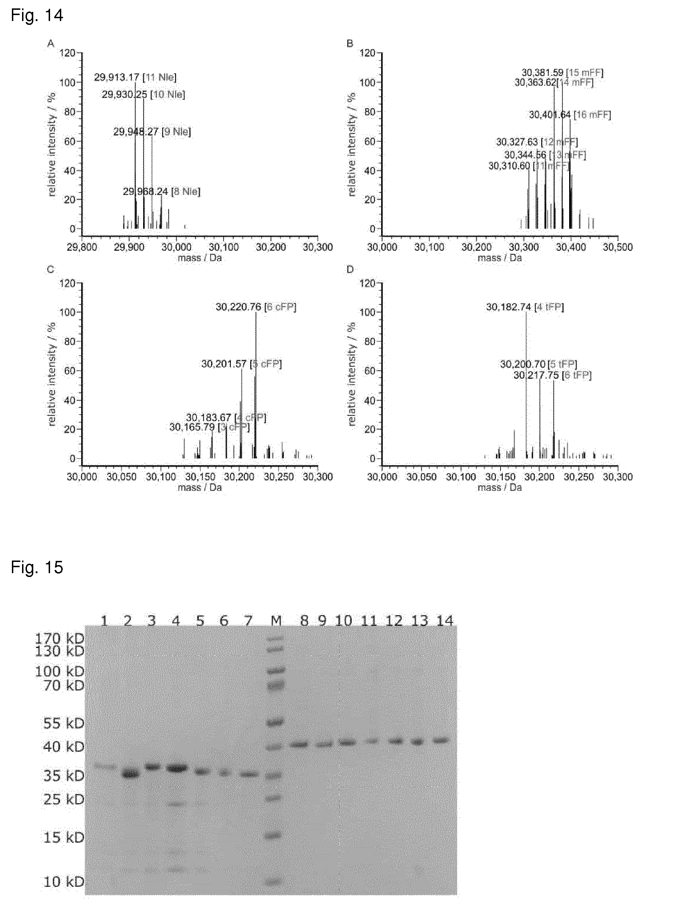

Fig. 14: Determination of the incorporation efficiency of Nle into TTL. LC-ESI MS-spectra of TTL[Nle] (A), TTL[mFF] (B), TTL[cFP] (C) and TTL[tFP] (D) produced by supplementation of the ncAA. The main peaks are labeled with the experimentally determined masses; the number of incorporated ncAA residues is shown in brackets.
   A: The main species corresponds to the TTL with all 11 Met exchanged by Nle.
   B: The main protein species corresponds to the TTL with all 15 Phe exchanged by mFF.
   C: The main species corresponds to the TTL with all 6 Pro exchanged by cFP.
   D: The main species corresponds to the TTL with all 4 Pro exchanged by tFP.
Fig. 15: Purified synthetic TTL and OYE variants. 4-12% Bis/Tris polyacrylamid gel; Comassie blue stain. M, prestained SDS ladder mix (5 µL); 1, TTL[Met]; 2, TTL[Nle]; 3, TTL[cFP]; 4, TTL[tFP]; 5, TTL[mFF]; 6, TTL[Nle] (biosynthesized Nle, D₆₀₀ 6); 7, TTL[Nle] (biosynthesized Nle, D₆₀₀ 6); 8, OYE[Met]; 9 OYE[Nle]; 10, OYE[cFP]; 11, OYE[tFP]; 12, OYE[mFF]; 13, OYE[Nle] (biosynthesized Nle, D₆₀₀ 6); 14, OYE[Nle] (biosynthesized Nle, D₆₀₀ 6).

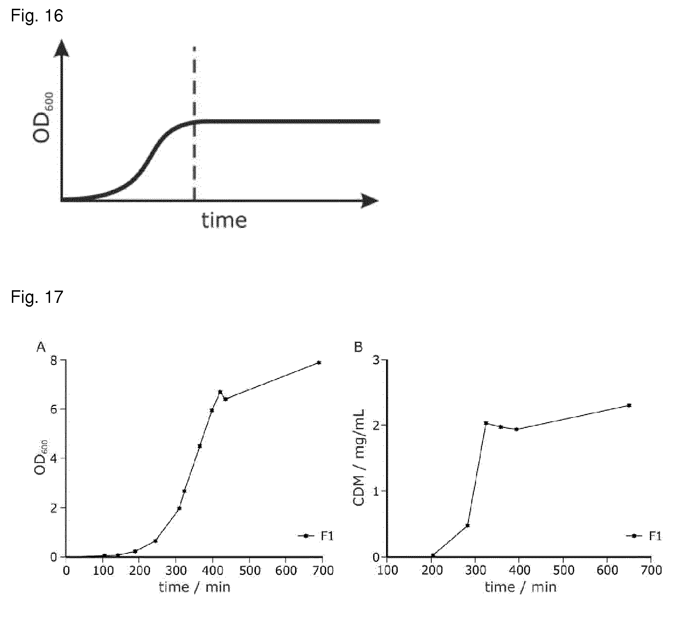

Fig. 16: Scheme of Nle biosynthesis in growing cells and incorporation into TTL.
Fig. 17: Bioreactor process analysis.
   A: Growth curves D₆₀₀ vs. time for bioreactor 1
   B: Growth curves CDM vs. time for bioreactor 1

### DETAILED DESCRIPTION OF THE INVENTION

The present invention provides methods for the production of proteins and/or polypeptides by cell culture. In particular, the invention provides an enhanced method for the production of a modified target polypeptide using at least one ncAA, wherein the host cells are cultured in a conventional medium containing a defined amount of a complex amino acid source until a precisely defined point in the growth curve of the cells which is defined by the depletion of a particular cAA. The particular cAA is then replaced by an ncAA. The ncAA is added to the culture media upon depletion of the particular cAA and no change of medium is needed.

To make the technique attractive for industrial protein engineering, it is important to develop a method for an inexpensive production of ncAAs as well as synthetic proteins in large scale.

Existing protocols for global ncAA incorporation use auxothrophic *E. coli* strains. The cells are first grown in medium containing all 20 canonical amino acids (synthetic minima medium or complex medium). As the cells reach the desired optical density for protein induction, they are sedimented and the supernatant is removed. The cell pellets are washed and then resuspended in minimal medium containing the desired ncAA instead of the respective ncAA, and protein expression is induced. An alternative technique uses minimal medium supplemented with limiting amounts of a particular cAA to support growth until the desired optical density for target protein induction is reached. Upon depletion of the cAA, the ncAA is added and target protein expression is induced.

NcAAs are a diverse group of compounds beyond the standard set prescribed by the genetic code. While the genetic code was evolutionarily established with only 20 cAAs, ncAAs are widespread in nature. More than 700 ncAAs are produced as secondary metabolites in fungi and plants. As well, ncAAs can be used as building blocks in non-ribosomal peptide synthesis, e.g., for the antibiotics micrococcin P1 and ramoplanin.

Due to their occurrence in nature, their biological properties and their amenability to ribosomal translation into proteins, ncAAs are interesting compounds for biotechnological applications. Many commercial ncAAs are very expensive and can be procured only in small amounts. For the production of synthetic proteins in the bioreactor, they must be available in sufficient amounts at a reasonable price.

Thus, the present invention relates to a method of biosynthesis of a ncAA as well as to a method of synthesis of a modified target polypeptide incorporated said biosynthesized ncAA.

The methods may include one or more ncAA selected from the group consisting of methoxinine, norleucine, azidonorleucine, homopropargylglycine, m-fluoro-phenylalanine, o-fluoro-phenylalanine, p-fluoro-phenylalanine, cis-hydroxy-proline, trans-hydroxy-proline, cis-fluoro-proline, trans-fluoro-proline, 4-aza-tryptophane, 4-fluoro-tryptophane, 7-fluoro-tryptophane, 4-amino-trypthophane, 4-thiofuran-tryptophane, m-fluoro-tyrosine, o-fluorotyrosine, and dopamine, azidohomoalanine, homopropargylglycine, p-bromophenylalanine, p-iodophenylalanine, azidophenylalanine, acetylphenylalanine, ethynylphenylalanine, (R)-2-amino-3-(4-ethynyl-1 H-pyrol-3-yl)propanoic acid, 1,2,4-triazole-3-alanine, 2-aminopheptanoic acid, 2-butynylglycine, 2-fluorohistidine, 2-pyridylalanine, 3-(1-naphthyl)alanine, 3-(2-naphthyl)alanine, 3-(2-thiazolyl)-DL-alanine, 3-(5-bromonindolyl)alanine, 3-(6-bromoindolyl)alanine, 3-(6-chloroindolyl)alanine, 3-bromo-tyrosine, 3-idiotyrosine, 3-methyl-L-histidine, 3-nitro-L-tyrosine, 3-pyridylalanine, 3-thienyl-alanine, 4-aminotryptophan, 4-fluorotryptophan, 4-propyl-L-tyrosine, 4-pyridylalanine, 5-ethynyl-tryptophan, 5-fluorotryptophan, 5-hydroxytryptophan, 6,6,6-trifluoro-2-aminohexanoic acid, 6-ethynyl-tryptophan, 6-fluorotryptophan, alkynephenylalanine, allylglycine, alpha-GalNAc-threonine, aminobutyric acid, aminooxyaceticacid, anisopropyl-L-phenylalanine, azidohomoalanine, azidonorleucine, benzofuranylalanine, beta-2-thienyl-L-alanine, beta-GlcNAc-serine, biocytin, cis-2-amino-4-hexenoic acid, cis-crotylglycine, diaminobutyric acid, dihydroxyl-L-phenylalanine, dihydroxy-phenylalanine, ethionine, fluorinated phenylalanine, hexafluoroleucine, homoallylglycine, homoglutamine, homoproparglyglycine, isopropyl-L-phenylalanine, L-methylhistidine, L-phosphoserine, m-acetyl-L-phenylalanine, m-allylphenylalanine, m-bromophenylalanine, m-ethynylphenylalanine, m-hydroxyphenylalanine, m-methoxy-L-phenylalanine, m-methoxyphenylalanine, m-methyl-phenylalanine, norleucine, norvaline, O-(2-propynyl)-L-tyrosine, O-4-allyl-L-tyrosine, O-allyl-L-tyrosine, o-bromophenylalanine, O-methyl-L-tyrosine, o-methyl-phenylalanine, O-propargyltyrosine, p-(3-oxobutanoyl)-L-phenylalanine, p-acetoacetylphenylalanine, p-acetyl-L-phenylalanine, p-acetylphenylalanine, p-acyl-L-phenylalanine, p-amino-L-phenylalanine, para-azidophenylalanine, para-cyanophenylalanine, para-ethynylphenylalanine, para-halophenylalanine, p-azido-L-phenylalanine, p-azidophenylalanine, p-benzoyl-L-phenylalanine, p-bromo-L-phenylalanine, p-bromophenylalanine, p-chlorophenylalanine, p-ethylphenylalanine, p-ethylthiocarbonyl-L-phenylalanine, p-ethynyl-phenylalanine, phosphonoserine, phosphonotyrosine, p-idiophenylalanine, p-iodophenylalanine, p-methyl-phenylalanine, p-nitro-L-phenylalanine, p-proparglyoxyphenylalanine, pxF, poxPhe, selenocysteine, selenomethionine, S-nitrosohomocysteine, telluromethionine, thia-proline, trans-2-amino-4-hexenoic acid, trans-crotylglycine, trifluoroisoleucine, trifluoroleucine, etc. Preferably the ncAA is norleucine or a tryptophan analogues.

The term "bioreactor" as used herein refers to any vessel used for the growth of a host cell culture. The bioreactor can be of any size so long as it is useful for the culturing of host cells. Typically, the bioreactor will be at least 1 liter and may be 10, 100, 250, 500, 1000, 2500, 5000, 8000, 10,000, 12,0000 liters or more, or any volume in between. The internal conditions of the bioreactor, including, but not limited to pH and temperature, are typically controlled during the culturing period. The bioreactor can be composed of any material that is suitable for holding host cell cultures suspended in media under the culture conditions of the present invention, including glass, plastic or metal. One of ordinary skill in the art will be aware of and will be able to choose suitable bioreactors for use in practicing the present invention.

To customize the production of synthetic proteins for biotechnology applications, high yield and low cost processes are desirable. Basically, two strategies can be followed to enhance protein production by an existing expression system. On the one hand, engineering the expression strain can increase protein production efficiency. On the other hand increased cell productivity realized by high cell density fermentation (HCDF) can raise total productivity of the desired end product. Major advantages of HCDF can be seen in reduced culture volumes, enhanced downstream processing, reduced wastewater lower production cost and last but not least, reduced investment in equipment.

The separation of the growth and the production phase as well as the right promoter system can increase productivity.

The term "cell density" as used herein refers to that number of cells present in a given volume of medium.

The terms "culture" and "cell culture" as used herein refer to a host cell population that is suspended in a medium (see definition of "medium" below) under conditions suitable to survival and/or growth of the cell population. As will be clear to those of ordinary skill in the art, these terms as used herein may refer to the combination comprising the host cell population and the medium in which the population is suspended.

The terms "medium", "cell culture medium", "culture medium", and "growth medium" as used herein refer to a solution containing nutrients which nourish growing host cells. Typically, these solutions provide essential and non-essential amino acids, vitamins, energy sources, lipids, and trace elements required by the cell for minimal growth and/or survival. The solution may also contain components that enhance growth and/or survival above the minimal rate, including hormones and growth factors. The solution is preferably formulated to a pH and salt concentration optimal for cell survival and proliferation. The medium may also be a "defined medium"- a serum-free medium that contains no proteins, hydrolysates or components of unknown composition. Defined media are free of animal-derived components and all components have a known chemical structure.

"Recombinantly expressed polypeptide" and "recombinant polypeptide" as used herein refer to a polypeptide expressed from a host cell that has been genetically engineered to express that polypeptide. The recombinantly expressed polypeptide can be identical or similar to polypeptides that are normally expressed in the host cell. The recombinantly expressed polypeptide can also be foreign to the host cell, i.e. heterologous to peptides normally expressed in the host cell.

As used herein, the phrases "polypeptide" or "polypeptide product" are synonymous with the terms "protein" and "protein product," respectively, and, as is generally understood in the art, refer to at least one chain of amino acids linked via sequential peptide bonds. In certain embodiments, a "modified target polypeptide" or the like is a protein encoded by an exogenous nucleic acid molecule that has been transformed into a host cell. In certain embodiments, wherein an exogenous DNA with which the host cell has been transformed codes for the "modified target polypeptide" the nucleic acid sequence of the exogenous DNA determines the sequence of amino acids. In certain embodiments, a" modified target polypeptide" is a protein encoded by a nucleic acid molecule that is endogenous to the host cell. In certain embodiments, expression of such an endogenous protein of interest is altered by transfecting a host cell with an exogenous nucleic acid molecule that may, for example, contain one or more regulatory sequences and/or encode a protein that enhances expression of the protein of interest.

Polypeptides that may desirably be expressed in accordance with the present invention will often be selected on the basis of an interesting biological or chemical activity. For example, the present invention may be employed to express any pharmaceutically or commercially relevant enzyme, receptor, antibody, hormone, regulatory factor, antigen, binding agent, etc.

The modified target polypeptides of interest may be produced in prokaryotes such as bacteria, or in lower eukaryotes such as yeast. One skilled in the art would know that the culture conditions for yeast and bacterial cell cultures will differ, and will understand how these conditions will need to be adjusted in order to optimize cell growth and/or protein production.

Host cells that may be used to practice certain embodiments of the present invention include auxotrophic host cells (whether prokaryotic or eukaryotic). Auxotrophic cells may exhibit single, double, triple, quadruple, or greater levels of auxotrophy (each level of auxotrophy indicates a particular organic compound, specifically a canonical amino acid, that the organism is unable to synthesize or otherwise lacks and must be supplied to the cell). Host cells may be genetically engineered to be auxotrophic for a certain cAA.

Suitable yeast strains for polypeptide production include *Saccharomyces cerevisiae, Schizosaccharomyces pombe, Pichia pastoris, Kluyveromyces* strains, *Candida,* or any yeast strain capable of expressing polypeptide of interest.

The establishment of ncAA incorporation as a new toolkit for protein engineering requires a method to produce synthetic proteins in large scales. Existing protocols for ncAA incorporation are performed at low cell densities (D₆₀₀ ∼ 1, mid-log). The present invention focused on the development of HCDF protocols appropriate for large-scale, high-yield SPI experiments in *E. coli.* An important aspect of the present invention is a new protocol for synthetic protein production in a bioreactor.

As the ncAA model amino acid, Nle was chosen. This amino acid is a side product of the branched chain amino acid biosynthesis. It was shown that global substitution of the Met residues in the thermophilic lipase TTL by Nle substantially improved the activity of the enzyme without otherwise indispensable heat pretreatment (M.G. Hoesl, et al., ChemCatChem, 2011. 3(1): 213-22). Thus, TTL was used as the model enzyme for the development of ICDF protocols for SPI in *E. coli.* In order to analyze whether global Met substitution by Nle would be beneficial for other enzymes of the alpha/beta-hydrolase class, synthetic Nle variants of the biotechnologically relevant enzymes of EC 3.1 were produced. In a further embodiment of the invention, biosynthesized Nle was incorporated *in situ* into TTL in the bioreactor.

As a further embodiment of the invention, ICDF procedures for the production of synthetic proteins in shake flask cultures as well as the bioreactor were developed. New Minimal Medium described in the literature for SPI in *E. coli* consists of 12 components (M.G. Hoesl, et al., ChemCatChem, 2011. 3(1): 213-22) which makes it tedious to prepare and rather expensive. Media for industrial scale fermentations should be as simple and inexpensive as possible. Especially the amino acids are problematic for ICDF procedures. Feeding solutions of fed-batch processes are usually highly concentrated and if an amino acid must be included, the solubility of most of these compounds is easily exceeded.

As a further embodiment of the present invention, a simple and low cost medium formulation was developed. Growth analysis showed that the reduced medium very well supported growth of the B834(DE3) strain and its descendants indicating that all essential nutrients were provided in satisfactory amounts by yeast extract supplementation.

The results of the titration experiment showed that the method is adequately precise to determine limiting amino acid concentrations that guarantee cAA depletion necessary for ncAA incorporation. The titration has to be performed for every new strain, as well as for each of the 20 cAAs. As well, slightly deviating amino acid compositions of different lots of YE have to be considered.

Compared to the shake flask the protocol could be up-scaled in a batch process by a 1 0-fold increase of the nutrients. A final D₆₀₀ of 30 was reached in the bioreactor. Protein expression was successful for shake flask, as well as bioreactor experiments.

The established high cell density SPI protocol for shake flask cultures was evaluated with the model enzyme TTL and 3 different hydrolases. For all 4 enzymes parent and synthetic variants were expressed as soluble proteins. The amounts of 20 - 30 mg/L for parent proteins, as well as half the amount for synthetic variants were achieved. It is possible that individual adaption of expression conditions can be helpful to increase protein yields.

An important aspect for SPI experiments is the choice of expression system. The cutinases were best expressed from the pMS470 vector carrying a trc promoter. This observation points out the importance of the expression system, specifically the promoter, for SPI.

Full Nle labeling was confirmed for all 3 cutinases by mass analysis, although detectable amounts of partially labeled proteins were found, too. This is most likely caused by incomplete depletion of Met.

The high cell density (D₆₀₀ 30) batch process for SPI of Nle provided in the medium yielded fully labeled TTL. This indicates that the necessary Met depletion can be determined simply by observing the growth arrest in late log phase. The batch process was highly reproducible and predictable.

In comparison to the parent protein, the TTL[Nle] variant showed increased activity on short chain esters para-nitrophenyl acetate and para-nitrophenyl butyrate.

It is one embodiment of the invention to biosynthesize Nle and simultaneously to incorporate it into a target protein in a bioreactor process.

### EXAMPLES

The Examples which follow are set forth to aid in the understanding of the invention but are not intended to, and should not be construed to limit the scope of the invention in any way. The Examples do not include detailed descriptions of conventional methods, e.g., cloning, transfection, and basic aspects of methods for overexpressing proteins in microbial host cells. Such methods are well known to those of ordinary skill in the art.

Synthetic variants of TTL, a lipase of a thermophile bacterium, the cutinases Cut1 and Cut2 from *Thermobifida cellulosilytica,* an esterase from *Thermobifida halotolerans* as well as Old Yellow Enzyme (OYE), a oxidoreductase from *Geobacillus kaustophilus* are used as model enzymes.

### Example 1 - High cell density SPI in E.coli

The methionine Met auxothrophic *E. coli* strain B834(DE3) was chosen as the host strain for SPI of norleucine (Nle). Strain B834(DE3) is a B strain carrying a mutation in the Met biosynthesis pathway which makes it dependent on exogenous Met supply in minimal medium.

Being a multi-component medium, New Minimal Medium (NMM) is badly customizable for high cell density fermentations (HCDF)(N. Budisa, et al., Eur J Biochem. 1995 Jun 1;230(2):788-96). In this experiment the original recipe of NMM was progressively changed to reduce the number of ingredients to the essential ones (see table 1 and table 2)

In NMM, the cells grow to a final D₆₀₀ of 2 and the depletion of the cAA is usually designed to occur at mid log (D₆₀₀ 1). As growth is not restored by the addition of the ncAA during target protein expression, the final cell numbers are comparably low (O₆₀₀ 1 vs. D₆₀₀ ≥4 for routine protein expression in LB medium). Lower cell numbers often yield less protein, therefore, a HCDF (final D₆₀₀ 3.6 in minimal medium) protocol in shake flasks was establish in order to produce high amounts of synthetic proteins.

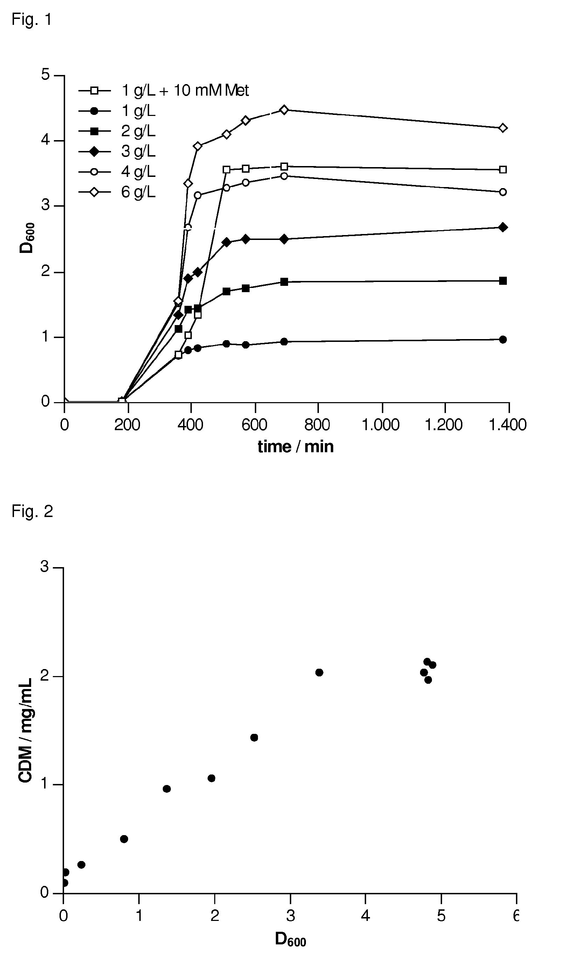

As outlined above, auxotrophic cells must be supplemented with the cAA in the medium in order to grow (first phase of SPI). Higher cell densities require more supplements and the water solubility especially of the aromatic amino acids is quite low. To overcome the problem of amino acid solubility for HCDFs the applicability of yeast extract (YE) as the Met source was evaluated. The titration experiment in Fig. 1 shows that YE is a suitable Met supply for the growth of B834(DE3). B834(DE3) transformed with an expression plasmid of TTL (pTTL) was used in order to evaluate the amount of YE necessary and sufficient to sustain growth and plasmid maintenance to a certain D₆₀₀. The culture medium was designed such that it contained enough glucose and other nutrients except Met for the cells to reach a final D₆₀₀ of 3.6. The medium was inoculated with the cells and 100 ml each in a shake flask were supplemented with a specific amount of YE as indicated in Fig. 1. The positive control contained 1 g/L YE and an excess of 10 mM Met. 1 mM Met per g CDM is determined to be sufficient for Met auxothrophic strains. The cultures were grown at 37°C and samples withdrawn for D₆₀₀ determination at repeated intervals over 24h. The results (Fig. 1) indicate a direct correlation between the amounts of YE added and the arrest of growth: As the amount of YE raises the cells grow to a higher D₆₀₀. Met is indeed the limiting factor in the growth medium: B834(DE3) supplemented with only 1 g/L YE grow to a final D₆₀₀ of ∼0.8 while they reach the expected maximal D₆₀₀ ∼3.5 if 10 mM Met is added in excess on top of 1 g/L YE.

Starvation of *E. coli* strains may influence the optical density. For that reason, the correlation between cell density and cell dry mass (CDM) was determined for the culture supplemented with 1 g/L YE and excess of Met. The results in Fig. 2 show that for this experiment a linear correlation between D₆₀₀ and CDM was obtained for D₆₀₀ values ≤3.6. The D₆₀₀ values >3.0 for the curve (white square) in Fig. 1 were corrected according to this correlation. For all further experiments the correlation between D₆₀₀ and CDM was linear for all further experiments. D₆₀₀ values >3.6 were obtained for the culture supplemented with 6 g/L of YE. This is most probably due to the introduction of extra carbon source by YE.

### Example 2 - Shake flask

The strain must be auxotrophic for Met to incorporate Met analogs, i.e. Nle, into target proteins. Every SPI experiment is separated in two phases. In phase I (growth phase) the auxotrophic strain is grown with a limiting concentration of Met. After Met depletion the ncAA Nle (analog culture) or Met (canonical culture) are added to the medium and protein expression is induced by IPTG (Phase II). The protocol can be adapted for other cAAs and their ncAA analogs.

### Phase I:

1 L of M9 medium containing YE as limiting Met source (see table 1) for Nle incorporation (analog culture) and Met control (canonical culture) in 2 L baffled flasks were prepared. Main cultures were inoculated out of a fresh ONC or with a 1 mL glycerol stock (D₆₀₀ 1) of strain B834(DE3) carrying the inducible expression plasmid. The cultures were incubated at 37°C with vigorous shaking until the D₆₀₀ remained constant for 30 minutes. The growth arrest indicated Met depletion.

**Table 1: Composition M9 medium**

| component | C_{final} | 1 L/D₆₀₀ 3.6 |
|---|---|---|
| M9 salts (Sigma) | 1 X | 200 mL |
| MgSO₄ | 0.1 g/g CDM | 1 mL |
| CaCl₂ | 1 µg/L | 1 mL |
| glucose2 | 3 g/g CDM | 20 mL |
| trace elements | 50 L/g CDM | 60 µL |
| antibiotic | 1 X | 1 mL |
| amino acid supplementation | 1 mM (∼0.13 g/g CDM) | 10 mL |
| yeast extract (YE) | 3.5 g/L | 3.5 g |
| ddH₂O | - | up to 1000 mL |

### Phase II:

The non-induced analog culture was supplemented with 1 mM of Nle, as well as the non-induced canonical culture with 1 mM Met. Expression of the target protein was induced by adding 0.4-1 mM IPTG to each culture and was performed for 4 h with vigorous shaking. The induction temperature was dependent on the target protein. The three cutinases were expressed at 28°C and the TTL at 30 °C. At the end of the induction the D₆₀₀ was read. Cells were harvested by low speed centrifugation (20 min, ∼4000 x g, 4°C) and resuspended in 5 mL Ni-chelate lysis buffer per gram cell wet weight (CWW). After 1 h incubation on ice, cells were directly used for protein purification or the suspensions were stored at -80 °C until use.

### Example 3 - Bioreactor

All experiments done in a bioreactor were performed with a DASGIP bioreactor system. The bioreactors were suitable for up to 1400 mL working volume and equipped with a pH and an oxygen electrode. For stirring, a 6-flat blade disc turbine was used. Basically, the medium formulation according to table 2 was used. The pH was pre-set to 7 and adjusted automatically with 10% (v/v) H₃PO₄ and 12.5% (v/v) NH₃. The partial pressure of oxygen (pO₂) was pre-set to a minimum concentration of dissolved oxygen of 30% and was regulated via the stirrer velocity.

**Table 2 Composition M9 medium adapted for the bioreactor**

| component | c_{stock} | V_{St[mL]}/1000 mL |
|---|---|---|
| M9 salt stock | 5 X | 200 |
| MgSO₄ | 1 M | 10 |
| Ca²⁺ / citric acid | 24 mg/mL | 10 |
| glucose | 1 M | 200 |
| trace elements | ∼18.7 X | 0.600 |
| yeast extract (YE) | - | 35 g |
| ddH₂O | - | up to 1000 ml |
| antifoam (1:10) | - | 1 |

### Example 4 - Production of Nle-labeled proteins in the bioreactor

The increased cell density fermentations (ICDF) protocol established in 1 L shake flask cultures was scaled up for the bioreactor. M9 adapted for the bioreactor (table 2) was used. The process was scaled up 1 0-fold compared to shake flask cultivations and was designed to yield a theoretical final D₆₀₀ of 36 (equals to 12 g cell dry mass, CDM). According to the theoretical amount of YE as Met source to lead to growth arrest at a D₆₀₀ of 30, 35 g/L YE were anticipated. In comparison to the shake flask experiment, all ingredients were added in a 1 0-fold access, except of the M9 salt and Ca²⁺ stocks. The Ca²⁺ stock contained 24 mg/mL CaCl₂ x 2H₂O and 120 mg/mL citric acid (molar ratio Ca²⁺ / citric acid is ~1) as a chelating agent to prevent precipitation of calcium phosphate in the medium adapted for the bioreactor. Each culture in a bioreactor was inoculated with 1 mL glycerol stock of B834(DE3) {pTTL} (D₆₀₀ ~1) and grown at 37°C to the point of Met depletion as indicated by the constant D₆₀₀ for 30 min. Then the bioreactor was cooled to 30°C and 20 mM of the amino acid (Met or Nle) were supplemented. The expression was induced by 1 mM IPTG and was performed for 4 h at a pH 7 and a pO₂ minimum of 30%. At the end of the bioreactor process, the cells were harvested by low speed centrifugation, resuspended in lysis buffer and stored at -80 °C.

### Example 5 - Nle production

M9 medium adapted for Nle production was used (table 2). The process was developed to be limited by Val/Ile supplementation (100 mg each) to yield a theoretical final D₆₀₀ of about 3 (equals to 1 g CDM). The bioreactor was inoculated with an overnight culture of BWEC10 (BL21 (DE3) Δ*ilvBN::0*, Δ*ilvIH::0 and* Δ*ilvGM::O*) to an D₆₀₀ of 0.01 and grown at 32°C to presumed Val depletion after 20 h as indicated by constant D₆₀₀. Then the bioreactor was kept at 32 °C for 52 h. At the end of the bioreactor process, the cells were harvested by low speed centrifugation, the supernatant was sterile filtered (0.45 µM), aliquoted in 50 mL falcons and stored at -80 °C.

### Example 6 - Production of synthetic proteins containing biosynthesized Nle

The medium was based on M9 medium adapted for the bioreactor (table 2), but slightly altered for each process. Details are summarized in table 3. All supplementations during the bioreactor process are shown in table 4.

**Table 3: Composition of media adapted for Nle production in a bioreactor**

| | | bioreactor 1 | bioreactor 2 | bioreactor 3 |
|---|---|---|---|---|
| component | Cₛₜ | volume/amount | volume/amount | volume/amount |
| M9 salts | 5 X | 200 mL | 200 mL | 200 mL |
| MgSO₄ | 1 M | 1 mL | 1 mL | 1 mL |
| Ca²⁺ | 1 mg/mL | 1 mL | 1 mL | 1 mL |
| glucose2 | 1 M | 55.6 mL | 111.1 mL | 111.1 mL |
| trace elements | ∼18.7 X | 60 µL | 180 µL | 180 µL |
| yeast extract | - | 7 g | 3.5 g | 3.5 g |
| ddH₂O | - | up to 1000 mL | up to 1000 mL | up to 1000 mL |
| ampicillin | 100 mg/mL | 1 mL | 1 mL | 1 mL |
| kanamycin | 50 mg/mL | 1 mL | 1 mL | 1 mL |
| antifoam | 1:10 | 1 mL | 1 mL | 1 mL |

**Table 4: Supplementations during the bioreactor process**

| | | bioreactor 1 | bioreactor 2 | bioreactor 3 |
|---|---|---|---|---|
| component | cst | volume/amount | volume/amount | volume/amount |
| Val | 100 mM | - | 5 mL | |
| Ile | 100 mM | - | 5 mL | 5 mL |
| Met | 100 mM | - | 5 mL | |
| glucose | 1 M | - | 50 mL | 50 mL |
| YE (3.5 g/L) | 60 g/L | - | - | 60 mL |

### Nle production in growing cells (bioreactor culture #1)

The process was designed to yield a theoretical final D₆₀₀ of 6 (equals 2 g CDM). Accordingly, 7 g/L YE were anticipated to lead to Met depletion at D₆₀₀ 6. The bioreactor was inoculated with BWEC14 (B834(DE3) Δ*ilvBN::0*, Δ*ilvIH::0 ΔilvGM::0 and metE)* {pTTL + pLeu*} to an initial D₆₀₀ of 0.01 and grown at 37°C to Met depletion as indicated by constant D₆₀₀ for 30 min. Then the bioreactor was cooled to 30°C and expression was induced by the addition of 1 mM IPTG. Expression was performed for 4 h at a pH 7 and a pO₂ minimum of 30%. At the end of the bioreactor process, the cells were harvested by low speed centrifugation, resuspended in lysis buffer and stored at -80 °C until further processing.

### Nle production in resting cells (bioreactor culture #2)

The process was designed to yield a theoretical final D₆₀₀ of 10 (equals to 3.3 g CDM). A two-step process was carried out: (1) Accumulation of cell mass up to D₆₀₀ 3 then depletion of Val and production of Nle (zero-growth phase); (2) growth restart by the addition of Val, further accumulation of cell mass until depletion of Met at D₆₀₀ 10; then induction of target protein production in the presence of Nle biosynthesized in the zero-growth phase. 3.5 g/L YE were anticipated to lead to Val depletion at D₆₀₀ 3. In order to ensure that Met was not depleted at the first growth arrest, 280 µM Met were supplemented to support growth until D₆₀₀ 10. The bioreactor was inoculated with BWEC14{pTTL} to an initial D₆₀₀ of 0.01 and grown at 37°C to Val depletion as indicated by a constant D₆₀₀. After depletion, a zero-growth period for Nle production was performed at 37°C, pH 7, pO₂ of 30%. 0.5 mM valine was added for growth restart to bioreactor 2, but the strain did not restore growth. All supplementations tested for growth restart were inefficient.

### Nle production in resting cells (bioreactor culture #3)

The process was designed as in bioreactor 2. In contrast to bioreactor 2, strain BWEC14{pTTL + pLeu*} was used. As supplementation with Ile and glucose did not promote growth after zero growth phase, yeast extract was added. Addition of YE restored growth and after reaching constant D₆₀₀ the bioreactor was cooled to 30°C and expression was induced by 1 mM IPTG and was performed for 4 h at a pH 7 and a pO₂ minimum of 30%. At the end of the bioreactor process, the cells were harvested by low speed centrifugation, resuspended in lysis buffer as before and stored at -80 °C until further processing.

### Example 7 - Synthetic TTL

TTL was used as the model protein to establish the ICDF protocol for SPI of ncAA, specifically Met analogs, into target proteins. For SPI experiments an appropriately inducible expression system is needed. Therefore, the TTL expression construct carrying a C-terminal H6-tag described by (M.G. Hoesl, et al., 2011) was reproduced by Gibson Assembly (D.G. Gibson, et al., Nat Methods. 2009 May; 6(5):343-5).

Expression in M9 medium in 100 mL shake flask cultures was performed to test whether the expression plasmid worked and the ICDF protocol could be used for Nle incorporation. The cultures grew to a final D₆₀₀ of 3. As Met should be depleted at this point 1 mM of Nle was added to the culture medium. A second culture was supplemented with 1 mM Met as a control. Protein expression was induced by adding 1 mM of IPTG as soon as the *E. coli* culture reached D₆₀₀ 1.Samples were taken before and after induction for SDS-PAGE analysis. The clear over-expression bands in the induced samples (gel in Fig. 3, lane i) indicate that both TTL variants were successfully over-expressed. The Met-variant and the Nle-variant showed different migration behavior on SDS-PAGE gels, indicating the successful incorporation of Nle into the target protein.

A photometric assay with para-nitro-phenyl acetate (pNP-acetate) and para-nitro-phenyl butyrate (pNP-butyrate) was chosen to detect the successfully expressed active TTL in a bioreactor. Results in Fig. 4 indicate that the two protein variants have different activity towards esterase substrates. The non-canonical TTL[Nle] variant was slightly more active.

### Example 8 - Incorporation of externally added Nle into TTL and OYE (shake flask)

The Met auxotrophic B834(DE3) was used for the incorporation of either Nle (Met analogs, white circle) or Met (black circle), into TTL *(Thermoanaerobacer thermohydrosulfuricus* lipase, blue) and OYE (Old Yellow Enzyme, black and white square) (Fig. 5).

All cultures showed the expected growth behavior as growth arrest of B834(DE3) occurred at an D₆₀₀ of 3 upon depletion of Met. As expected the cultures supplemented with Met showed growth after induction, in contrast the cultures supplemented with Nle showed a reduced linear growth behavior. This is in agreement with the notion that the strain should not be able to grow on Nle in the absence of Met.

All values were determined in triplicates. Mean values are shown and the error bars indicate the deviation from the minimal and maximal measured values.

### Example 9 - Incorporation of externally added Nle into TTL and OYE (shake flask)

The attenuance at 600 nm (D₆₀₀) vs. time is shown. The Met auxotrophic B834(DE3) was used for the incorporation of either Nle (Met analogs, white circle) or Met (black circle), into TTL (Thermoanaerobacer thermohydrosulfuricus lipase, blue) and OYE (Old Yellow Enzyme, green).

All cultures showed the expected growth behavior as growth arrest of B834(DE3) occurred at an D₆₀₀ of 3 upon depletion of Met. As expected the cultures supplemented with Met showed growth after induction, in contrast the cultures supplemented with Nle showed a reduced linear growth behavior. This is in agreement with the notion that the strain should not be able to grow on Nle in the absence of Met.

All values were determined in triplicates. Mean values are shown and the error bars indicate the deviation from the minimal and maximal measured values (see Fig. 5)

### A: Expression of TTL[Met] and TTL[Nle]

Overexpression of TTL[Met] is clearly visible by an intense band at the calculated molecular weight of 30 kD (black arrow) in the induced sample. A comparable band is absent from the non-induced sample. Overexpression of TTL[Nle] is less clearly visible as the band runs lower than expected (25 kD, red arrow) and is blurred. The Met-variant and the Nle-variant showed different migration behavior in SDS-PAGE as reported previously [1].

### B: Expression of OYE[Met] and OYE[Nle]

Overexpression of OYE[Met] as well as OYE[Nle] is clearly visible at the calculated molecular weight size of 37 kD (black arrow) in the respective induced samples (see Fig. 6).

### Example 10 - Incorporation of biosynthesized Nle into TTL and OYE (bioreactor)

Unless otherwise indicated, the BWEC14 strain, capable of biosynthesizing Nle, was carrying the pLeuA* overexpression vector and the appropriate expression plasmid, either pQE80L-TTL-h6 (A, B) or pEamTA-OYE (C, D). Met depletion occurred as calculated at either D₆₀₀ 6 or D₆₀₀ 30. In case of the D₆₀₀ 6 process two different setups were run, one without and one with a nutrient feed (+ feed) during induction (see Fig. 7).
A: Growth curves D₆₀₀ vs. time for BWEC14 {pQE80L-TTL-h6}.
B: Growth curves cell dry mass (CDM) vs. time for BWEC14 {pQE80L-TTL-h6}.
   Controls for a non-induced culture (D₆₀₀ 6 non-induced), BWEC14 carrying the empty plasmid (pQE80L) and BWEC14 carrying the empty, as well as the pLeuA* overexpression plasmid (D₆₀₀ 6 pQE80L + pLeuA*) are also shown.
C: Growth curves D₆₀₀ vs. time for BWEC14 {pEamTA-OYE}.
D: Growth curves CDM vs. time for BWEC14 {pEamTA-OYE}.

The following controls are also shown: D₆₀₀ 6 +L-Met, supplemeThe following controls are also shown: D₆₀₀ 6 +L-Met, supplementation with L-Met at induction, no additional nutrient feed; D₆₀₀ 6 +feed +L-Met, supplementation with L-Met at induction, with additional nutrient feed; D₆₀₀ 6 +als + pLeuA*, B834(DE3) strain carrying the pLeuA* overexpression plasmid, but lacking Als (acetolactate synthase).

All cultures showed growth arrest due to Met depletion at the expected D₆₀₀ of either 6 or 30. Slight linear growth after induction occurred.

An overexpression band for the OYE[Nle] is clearly visible at the calculated molecular weight of 37 kD (orange mark), as well as for TTL[Nle] at the expected size of 25 kD (red mark) (see Fig. 8).

This implies that the biosynthesis of Nle and in-situ incorporation is possible when using a genetically engineered Met auxotrophic E. coli strain.

A Tetra-His antibody (Quiagen) was used as the primary and a goat anti-mouse 8g1 HRP conjugate as the secondary antibody. The membrane was incubated with SuperSignal West Pico Substrate for 5 minutes and the luminescence was detected using Gbox (Syngene) (see Fig. 9).
The expression of TTL[Nle] was not visible on an SDS gel unless the bioprocess was supplemented with an additional nutrient feed during protein expression. In order to confirm expression of TTL[Nle] a Western Blot was performed.

LC-ESI MS-spectra of TTL[Nle] produced by the incorporation of biosynthesized Nle at D₆₀₀ 6 without feed (A), D₆₀₀ 6 with feed (B) and D₆₀₀ 30 (C) are shown (Fig. 10).

The main peak species were labeled with the experimentally determined masses; the number of incorporated Nle residues is shown in brackets.

In all 3 cases the main species corresponds to the TTL with all 11 Met exchanged by Nle. Minor peaks indicating incompletely labeled subspecies were detected as well.

The incorporation efficiency corresponds to previously published data.

The productivity of the two ncAA Nle (black and grey square) and Nva (black and grey diamond) was followed over time (Fig. 11). The D₆₀₀ (white circles) is shown to demonstrate that Nle and Nva were produced during growth and not in resting cells, (E. Sycheva, T. et al., Microbiology, 2007. 76(6): p. 712-718). Each bioreactor culture reached the calculated D₆₀₀ of 6 (A, B, D and E) or 30 (C). In case of the D₆₀₀ 6 process two different setups, one without (A) and one with an additional nutrient feed (B) during induction, were run.

Unless otherwise indicated the BWEC14 strain capable of biosynthesizing Nle carried the pLeuA* overexpression and the appropriate expression plasmid, either pQE80L-TTL-h6 or pEamTA-OYE.
A: About 700 mg/mL of Nle were accumulated until the end of the bioreactor experiment, and even 400 mg/L had been produced during the growth phase until induction of target protein expression at t∼400 minutes.
B: The culture was fed with additional nutrients during the induction phase. The levels of Nle at induction (400 mg) and at the end of the process (800 mg/L) were slightly increased compared to A.
C: The increased cell densities led to a tremendous increase in Nle productivity. About 4 g/L were produced until induction of target protein expression (t∼800 min) and even up to ∼6 g/L of Nle were accumulated until the end of the process at 2000 minutes.
D: The productivity of Nle is not affected by the plasmid for target protein expression. Comparable results to A were obtained.
E: Productivity of B834(DE3) carrying the pLeuA* overexpression plasmid and the plasmid for target protein expression. Compared to the BWEC14 strain, B834(DE3) carries functional acetolactate synthase (als). It has been shown that *als* deficient strains show higher Nle productivity, especially when the pLeu operon is overexpressed [2]. The strain is capable to produce sufficient amounts of Nle until induction, even though compared to the als- strains, no accumulation of Nle during the expression is observed, indicating that Nle is introduced into the target protein.

### Example 11 - Incorporation of externally added Met, Pro and Phe analogs into TTL and OYE (bioreactor)

The Met auxotrophic strain B834(DE3), the Pro auxotrophic strain BWEC44 and the Phe auxotrophic strain BWEC23 were used for the incorporation of either Nle (Met analog), cFP/tFP (Pro analogs) or mFF (Phe analog) (see Fig. 12).
A: Growth curves D₆₀₀ vs. time for the strains carrying the pQE80L-TTL-h6 expression plasmid.
B: Growth curves CDM vs. time for the strains carrying the pQE80L-TTL-h6 expression plasmid.
C: Growth curves D₆₀₀ vs. time for the strains carrying the pEamTA-OYE expression plasmid.
D: Growth curves CDM vs. time for the strains carrying the pEamTA-OYE expression plasmid.

All cultures showed the expected growth behavior upon depletion of the respective cAA; B834(DE3) (TTL[Nle]+OYE[Nle]) and BWEC44 (TTL[cFP]+OYE[cFP]) arrested growth at D₆₀₀ 30; BWEC23 (TTL[mFF]+OYE[mFF]) at D₆₀₀ 20. In case of the expression of TTL[FP] and OYE[FP]The D₆₀₀, as well as the CDM, remained constant during the course of protein expression during 36 hours. However, in the other cultures (TTL[Nle], OYE[Nle], TTL[mFF] and OYE[mFF]) D₆₀₀ as well as CDM decreased with time which may indicate cell death.

All values were determined in triplicates. The cultures for the incorporation of cFP and tFP showed comparable growth behavior, therefore, the curves for tFP incorporation are omitted for clarity.

10% SDS polyacrylamid gel; Comassie blue stain. M, prestained SDS ladder mix (5 µL); ni, non-induced; i, induced after 4 h, 14 h and 24 h (see Fig. 13).
A: An overexpression band for OYE[cFP] and OYE[tFP] is visible at the expected size of 37 kD (white mark).
B: An overexpression band for TTL[cFP] and TTL[tFP] is visible at the expected size of 30 kD (black mark).
C: An overexpression band for the OYE[Nle] is clearly visible at the expected size of 37 kD (white mark), as well as for TTL[Nle] at 25 kD (black mark) as expected.
D: An overexpression band for the OYE[mFFe] is clearly visible at expected size of 37 kD (white mark), as well as for TTL[Nle] at 30 kD (black mark) as calculated.

To show the broad applicability of the method, different synthetic variants of two enzymes has been produced in the bioreactor. Auxotrophic strains for the cAA that should be exchange were grown until cAA depended growth arrest at D₆₀₀ of 30, supplemented with the ncAA and target protein expression was induced. The production of synthetic proteins in the bioreactor is suitable, by simply up-scaling the shake flask protocol.

LC-ESI MS-spectra of TTL[Nle] (A), TTL[mFF] (B), TTL[cFP] (C) and TTL[tFP] (D) produced by supplementation of the ncAA (see Fig. 14). The main peaks are labeled with the experimentally determined masses; the number of incorporated ncAA residues is shown in brackets.
A: The main species corresponds to the TTL with all 11 Met exchanged by Nle. The results are comparable to the results obtained for the incorporation of biosynthesized Nle though the incompletely labeled protein species are also detectable and were more prominent than in the experiment with biosynthesized Nle (see above).
B: The main protein species corresponds to the TTL with all 15 Phe exchanged by mFF. The fully labeled species was also detected (16 mFF). The pattern of incompletely labeled subspecies is comparable to published data.
C: The main species corresponds to the TTL with all 6 Pro exchanged by cFP. Minor subspecies are also detectable.
D: The main species corresponds to the TTL with all 4 Pro exchanged by tFP. The fully labeled species was detected, yet was less prominent than the fully labeled.

The obtained incorporation efficiencies are similar or higher compared to already published shake flask data (M.G. Hoesl, C.G. et al., ChemCatChem, 2011. 3(1): p. 213-221]).

4-12% Bis/Tris polyacrylamid gel; Comassie blue stain. M, prestained SDS ladder mix (5 µL); 1, TTL[Met]; 2, TTL[Nle]; 3, TTL[cFP]; 4, TTL[tFP]; 5, TTL[mFF]; 6, TTL[Nle] (biosynthesized Nle, D₆₀₀ 6); 7, TTL[Nle] (biosynthesized Nle, D₆₀₀ 6); 8, OYE[Met]; 9 OYE[Nle]; 10, OYE[cFP]; 11, OYE[tFP]; 12, OYE[mFF]; 13, OYE[Nle] (biosynthesized Nle, D₆₀₀ 6); 14, OYE[Nle] (biosynthesized Nle, D₆₀₀ 6) (see Fig. 15).

Synthetic TTL variants showed different migration behavior on SDS-PAGE gels as reported in previous publications [1].

### Example 12 - Nle biosynthesis in growing cells

A one step protocol for Nle production in growth phase was applied to determine if the co-transformed over-expression plasmid pLeu* is sufficient to shift the productivity of BWEC14 in direction of the ncAA Nle (scheme shown in Fig. 16). The analysis of D₆₀₀ and CDM in Fig. 14 shows that the expected D₆₀₀ of 6, corresponding to 2 g/L CDM, was reached. Growth stop due to Met depletion occurred after 400 minutes. Growth after induction was marginal. This can also be ascribed to the fact that after induction no further amino acid source was added to the Ile, Leu and Val auxothrophic BWEC14 strain.

The exponential growth phase up to D₆₀₀ 6 was sufficient to produce enough Nle for the standard SPI protocol as indicated by the red dotted line (100 mg/L). The branched chain amino acid concentrations decreased as expected. At the point of induction, all three amino acids were still present, although the free amount of Ile and Val was relatively low. Growth rates are comparable to previous bioreactor experiments with YE as the amino acid source

## Claims

1. An enhanced method for producing a modified target polypeptide containing at least one non-canonical amino acid (ncAA) comprising the steps of:
a) providing a host cell, the host cell comprising a vector encoding a target polypeptide;
b) cultivating said host cell in a medium comprising a complex amino acid source with a limiting amount of at least one canonical amino acid (cAA), whereupon said host cell logarithmically grows until depletion of said cAA;
c) adding a corresponding ncAA to said medium and expressing the target protein, whereupon the ncAA is incorporated into the target polypeptide; and
d) isolating the modified target polypeptide.

2. The method according to claim 1, wherein the host cell is a bacterial cell, preferably from the *Enterobacteriaceae* family, more preferred an *Escherichia* cell.

3. The method according to claim 1 or 2, wherein the host cell is auxotroph for at least one canonical amino acid.

4. The method according to any one of claims 1 to 3, wherein the complex amino acid source is a hydrolysate of protein material that is rich in amino acids, nitrogen compounds, carbohydrate and vitamins, such as yeast extract, casamino acids, tryptone, preferably it is a yeast extract.

5. The method according to any one of claims 1 to 4, wherein the complex amino acid source is added to a minimal medium.

6. The method according to any one of claims 1 to 5, wherein said ncAA is selected from the group consisting of a methionine analog, a tryptophane analog and trypsine analog, preferably from the group consisting of methoxinine, norleucine, homopropargylglycine, meta-fluoro-phenylalanine, ortho-fluoro-phenylalanine, para-fluoro-phenylalanine, cis-hydroxy-proline, trans-hydroxy-proline, cis-fluoro-proline, trans-fluoro-proline, 4-aza-tryptophane, 4-fluoro-tryptophane, 7-fluoro-tryptophane, 4-amino-trypthophane, 4-thiofuran-tryptophane, meta-fluoro-tyrosine, ortho-fluorotyrosine, dopamine, more preferably the ncAA is norleucine or a tryptophane analog.

7. The method according to any one of claims 1 to 6, wherein the modified target polypeptide is selected from the group consisting of antibodies, antibody fragments, enzymes, receptors, hormones, regulatory factors, growth factors, antigens, membrane proteins and binding agents.

8. The method according to any one of claims 1 to 7, wherein the method is carried out in a bioreactor.

9. The method according to claim 8, wherein the bioreactor is a large-scale bioreactor.

10. An enhanced method for substitution of a canonical amino acid by a ncAA during translation of a target polypeptide in a host cell, comprising culturing said cell in minimal growth medium and a defined amount of a complex amino acid source until a defined point in the growth curve, then adding the desired ncAA directly to the culture media, whereupon the ncAA is incorporated into the target polypeptide.

11. The method according to claim 10, wherein the complex amino acid source is a hydrolysate of protein material that is rich in amino acids, nitrogen compounds, carbohydrate and vitamins, such as yeast extract, casamino acids, tryptone, preferably it is a yeast extract.

12. The method according to claim 11, wherein the yeast extract concentration is in the range of about 0.5 to 10 g/L, preferably in the range of about 1 to 8 g/L, more preferred in the range of about 2 to 6 g/L.

13. An enhanced method for substitution of a canonical amino acid by a ncAA during translation of a target polypeptide in a host cell, comprising
a) providing a genetically engineered host cell which is capable of producing the ncAA, said cell comprising a vector encoding the target polypeptide, and
b) culturing said cell in minimal growth media and a defined amount of complex amino acids source whereupon the ncAA is biosynthesized, and
c) inducing expression of the target polypeptide, whereupon the biosynthesized ncAA is incorporated into the target polypeptide, and
d) isolating the target polypeptide.

14. A method for designing a growth medium for producing a modified target polypeptide comprising:
a) adding defined amounts of a complex amino acids source to a minimal medium;
b) growing a host cell in said medium;
c) measuring the D₆₀₀ of said medium at repeated intervals over a time period;
d) correlating the amounts of the complex amino acids source added and the arrest of growth;
e) supplementing the minimal medium with the determined amount of the complex amino acids source.
